# EUROPEAN PATENT SPECIFICATION

(11) **EP 3 127 543 B1**
(45) Date of publication and mention of the grant of the patent: **01.08.2018**
(21) Application number: 16175803.2
(22) Date of filing: 22.06.2016
(51) Int. Cl.: A61K 31/702, A61P 27/02, A61P 27/04

(54) **OPHTHALMIC FORMULATIONS COMPRISING OLIGOSACCHARIDES**
OPHTHALMISCHE FORMULIERUNGEN MIT OLIGOSACCHARIDEN
FORMULATIONS OPHTALMIQUES COMPRENANT DES OLIGOSACCHARIDES

(30) Priority: 03.08.2015 IT UB20152818
(43) Date of publication of application: 08.02.2017
(73) Proprietor: Consorzio Universitario Unifarm, 95125 Catania (IT)
(72) Inventor: BUCOLO, Claudio, 95125 Catania (IT); MUSUMECI, Salvatore, 95125 Catania (IT); MUSUMECI, Maria, 95125 Catania (IT); DRAGO, Filippo, 95125 Catania (IT)
(74) Representative: Bianchetti Bracco Minoja S.r.l.

(56) References cited:
- EP-A1- 0 765 666
- WO-A2-2005/055944
- P. CHATURVEDI ET AL: "Fucosylated human milk oligosaccharides vary between individuals and over the course of lactation", GLYCOBIOLOGY, vol. 11, no. 5, 1 May 2001 (2001-05-01), pages 365-372, XP055164028, ISSN: 0959-6658, DOI: 10.1093/glycob/11.5.365
- MYRIAM COULET ET AL: "Pre-clinical safety evaluation of the synthetic human milk, nature-identical, oligosaccharide 2'-O-Fucosyllactose (2'FL)", REGULATORY TOXICOLOGY AND PHARMACOLOGY, vol. 68, no. 1, 1 February 2014 (2014-02-01), pages 59-69, XP055256464, US ISSN: 0273-2300, DOI: 10.1016/j.yrtph.2013.11.005

## Description

The present invention relates to ophthalmic formulations comprising oligosaccharides present in colostrum and milk, in particular 2'-fucosyl-lactose and optionally polymers such as hyaluronic acid, which are useful for the treatment of eye surface disorders such as dry eye syndrome and/or eye tissue inflammations.

### Background

"Dry eye" syndrome, also known as keratoconjunctivitis sicca, is a multifactorial disease of the tears and the eye surface. According to the Definition and Classification Subcommittee of the International Dry Eye Work Shop (DEWS; www.theocularsurface.com) Dry eye is a multifactorial disease of the tears and ocular surface that results in symptoms of discomfort, visual disturbance, and tear film instability with potential damage to the ocular surface. It is accompanied by increased osmolarity of the tear film and inflammation of the ocular surface. The most common symptoms of dry eye syndrome include dryness, irritation, foreign body sensation, sensitivity to light, and itching. It is estimated that in the USA alone, nearly 5 million people over 50 years old have dry eye syndrome or keratoconjunctivitis sicca, or have had at least one episode of tear film alteration; about two-thirds of them are women (Smith et al., 2007). The prevalence of dry eye increases dramatically with age. The pathogenesis of dry eye is not entirely clear; however, it is believed that hyperosmolarity may affect the surface tension and stability of the tear film (Liu et al., 2009). Hyperosmolarity is an important characteristic of tears, which has been extensively studied, and is one of the most important markers for dry eye (Lemp et al., 2007). The hyperosmotic environment may contribute to triggering an inflammatory process; inflammation plays a leading part in the development and amplification of the signs and symptoms of dry eye syndrome.

The oligosaccharides present in human milk are mainly fucosylated. Of the fucosylated oligosaccharides in milk, 2'-fucosyl-lactose is the one present in the highest percentage in breast milk, and the first to be present in colostrum (Newburg, 1996; Newburg et al., 2004). Although the role played by oligosaccharides was initially unclear, it has recently been demonstrated that the oligosaccharides present in milk stimulate the immune system and protect breastfed babies against a variety of pathogens (e.g. viruses and bacteria), especially during the first 6 months (Coyne et al., 2005). It is also known that intestinal cells are covered with fucosylated glycoproteins and glycolipids that protect the body against pathogens (Coyne et al., 2005). Sarkar et al. (1996) identified oligosaccharide binding sites. It is interesting that 2'-fucosyl-lactose is the main oligosaccharide present in human colostrum (Musumeci et al., 2006).

Okano et al. recently demonstrated that fucosylated sugars induce a Th2 response (Okano et al., 2001). It was recently demonstrated that 2'-fucosyl-lactose possesses immunomodulating effects (Sotgiu et al., 2006). Chaumeil et al. (1994) postulated that bovine colostrum could be used to treat severe dry eye.

### Description of the invention

It has now been found that 2'-fucosyl lactose, applied topically to the eye in a suitable formulation, protects the eye surface by stabilising the tear film. Said effect has been demonstrated in an experimental model of dry eye in the rabbit, as indicated in the examples.

The object of the invention is therefore ophthalmic formulations comprising one or more oligosaccharides present in human colostrum or milk, especially 2'-fucosyl-lactose as specified in the claims. The formulations according to the invention can also optionally include hyaluronic acid and salts thereof.

The effective concentration of 2'-fucosyl lactose ranges from 0.001 to 5.0% (w/v), preferably from 0.01 to 2.0% (w/v).

The compositions according to the invention can take the form of ophthalmic solutions, ophthalmic suspensions, gels, lotions, liposomal forms, eyedrops, or other types of solid or semisolid forms, as well as medical devices for ocular use. The compositions may contain other ingredients such as surfactants, osmolarity-adjusting agents, buffers, preservatives, anti-oxidants, surfactants, complexing agents, viscosity-controlling agents and co-solvents. Sodium chloride, potassium chloride, magnesium chloride, calcium chloride, dextrose and/or mannitol can be used, for example, as osmolarity-adjusting agents. The final formulation will generally have an osmolarity compatible with the eye tissues (around 150-450 mOsm, preferably 250-350 mOsm).

Examples of suitable buffers include a phosphate, acetate, citrate or borate buffer, to maintain a suitable pH (6.0-7.5). The formulations can be presented in multi-dose or single-dose packs. In the case of multi-dose packs, preservatives such as thimerosal, ethanol, chlorobutanol, parabens, sodium edetate, sorbic acid, polyquaternium-1, or other conventional agents are preferably used. Said preservatives are generally used in concentrations ranging between 0.001 and 5.0% w/v. The single-dose packs are sterile, and do not usually contain preservatives.

Anti-oxidants, polymers and stabilisers in concentrations ranging from 0.001 to about 5% (preferably from 0.01% to 2%) can also be used.

Examples of surfactants include polysorbate 80 (polyoxyethylene sorbitan monooleate), polysorbate 60 (polyoxyethylene sorbitan monostearate), polysorbate 20 (polyoxyethylene sorbitan monolaurate) (commonly called Tween 80®, Tween® 60 and Tween® 20), poloxamers (Pluronic®; e.g., Pluronic® F127 or Pluronic® F108) or poloxamines (Tetronic®; e.g. Tetronic® 1508 or Tetronic® 908, Brij®, Myrj®, and other agents such as oleyl alcohol, stearyl alcohol, myristyl alcohol and docosohexanoyl alcohol, described in Martindale, 34th ed., pp. 1411-1416 (Martindale, "The Complete Drug Reference," S.C. Sweetman (Ed.), Pharmaceutical Press, London, 2005), and in Remington, "The Science and Practice of Pharmacy," 21st Ed., p. 291. The concentration of said compounds can range from 0.001 to 5%.

Examples of complexing agents include cyclodextrins, in particular hydroxypropyl-beta-cyclodextrin.

Examples of viscosity-controlling agents include hyaluronic acid, cellulose, polyvinylpyrrolidone and gellans.

The formulations of the invention are effective in the treatment of dry eye syndrome, tear film alterations and eye tissue inflammations, for contact lens wearers, and as adjuvant in antibiotic treatments of the eye region.

The invention is illustrated in detail in the following examples:

### Example 1

| | |
|---|---|
| 2'-fucosyl-lactose | 0.1 g |
| Hyaluronic acid | 0.1 g |
| Sodium dihydrogen phosphate | 0.2 g |
| Sodium chloride | 0.8 g |
| Benzalkonium chloride | 0.01 g |
| Purified water | q.s. to 100 mL |

### Example 2

| | |
|---|---|
| 2'-fucosyl-lactose | 0.2 g |
| Hyaluronic acid | 0.05 g |
| Sodium dihydrogen phosphate | 0.2 g |
| Sodium chloride | 0.8 g |
| Benzalkonium chloride | 0.01 g |
| Purified water | q.s. to 100 mL |

### Example 3

| | |
|---|---|
| 2'-fucosyl-lactose | 0.01 g |
| Hyaluronic acid | 0.01 g |
| Sodium dihydrogen phosphate | 0.2 g |
| Sodium chloride | 0.8 g |
| Benzalkonium chloride | 0.01 g |
| Purified water | q.s. to 100 mL |

### Example 4

| | |
|---|---|
| 2'-fucosyl-lactose | 0.01 g |
| Hyaluronic acid | 0.1 g |
| Sodium dihydrogen phosphate | 0.2 g |
| Sodium chloride | 0.8 g |
| Benzalkonium chloride | 0.01 g |
| Purified water | q.s. to 100 mL |

### Example 5

| | |
|---|---|
| 2'-fucosyl-lactose | 0.01 g |
| Sodium dihydrogen phosphate | 0.2 g |
| Sodium chloride | 0.8 g |
| Benzalkonium chloride | 0.01 g |
| Purified water | q.s. to 100 mL |

### Example 6

| | |
|---|---|
| 2'-fucosyl-lactose | 0.1 g |
| Sodium dihydrogen phosphate | 0.2 g |
| Sodium chloride | 0.8 g |
| Benzalkonium chloride | 0.01 g |
| Purified water | q.s. to 100 mL |

### Example 7

| | |
|---|---|
| 2'-fucosyl-lactose | 1 g |
| Sodium dihydrogen phosphate | 0.2 g |
| Sodium chloride | 0.8 g |
| Benzalkonium chloride | 0.01 g |
| Purified water | q.s. to 100 mL |

### Example 8 - Pharmacological tests

### Animals

New Zealand albino rabbits (1.8-2.0 kg) were used. All the animals were treated according to the guidelines of the Association for Research in Vision and Ophthalmology (ARVO).

### Model

A previously validated experimental model of "dry eye" (Shafiee et al., 2011) was used. The following parameters were evaluated: tear volume using Schirmer's test, tear break-up time (TBUT) and corneal integrity. An evaluation was also conducted with a slit lamp to determine the state of the eye tissues. The above-mentioned observations and parameters were performed 24 hours before and 24 hours after experimental dry eye was induced by treatment with atropine (Shafiee et al., 2011). Atropine pharmacologically inhibits the muscarinic receptors in the eye by reducing the production of the aqueous component of the tears, leading to an alteration in the tear film. Atropine sulphate (1.0% ophthalmic solution; Atropine Lux, Allergan, Italy) was instilled into the conjunctival sac of each eye three times a day. Fifteen minutes after the last administration of atropine, the ophthalmic formulation containing 2'-fucosyl-lactose was instilled. Schirmer's test was conducted by positioning the strips in the lower sac for 60 seconds, and measuring the wet strip (mm) as tear volume index. The TBUT was determined after instillation of 5 microlitres of 2% fluorescein sodium in sterile PBS on the lower eyelid, using the slit lamp (Sbisà, Florence, Italy) with a blue filter. The procedure was conducted three times consecutively, and the mean of the three readings was calculated. Corneal staining was evaluated with the National Eye Institute's classification system (Lemp, 1995). In addition to TBUT, fluorescein staining and Schirmer's test, the osmolarity of the tears (mOsm/L) was determined with an osmometer (Osmomat 30, Gonotech, Berlin, Germany) (Aragona et al., 2002). All data are expressed as mean ± S.D. unless otherwise indicated. The statistical analysis was conducted with ANOVA followed by Dunnett's test. A value of p<0.05 was considered significant.

### Results

The action of atropine is to induce pharmacological inhibition of the muscarinic receptors in the lacrimal gland, and therefore to reduce aqueous production and modify the stability of the tear film. Repeated topical administration of atropine followed by the carrier significantly reduced the tear volume
(3.75 ± 0.5 mm) compared with the baseline value (12.5 ± 0.5 mm) after 24 hours (Table 1). 2'-fucosyl lactose (0.01%, 0.1% and 1.0%) significantly inhibited the reduction in tear volume induced by atropine after 24 hours to a dose-dependent extent (Table 1). The maximum dose of 2'-fucosyl-lactose maintained the tear volume close to basal levels. These findings indicate that the treatments completely prevented atropine-induced effects on tear volume.

The eyes treated with the carrier alone (without 2'-fucosyl-lactose) showed a statistically significant reduction in terms of TBUT after 24 hours (reduction of TBUT 4.75 seconds) compared with the baseline TBUT (34.5 seconds). 2'-fucosyl-lactose (0.01%, 0.1% and 1.0%) significantly inhibited the reduction of TBUT induced by atropine after 24 hours, to a dose-dependent extent (Table 1). The eyedrops based on 2'-fucosyl-lactose (1%) maintained the TBUT values at physiological levels (Table 1). Schirmer's test indicates that topical treatment with 2'-fucosyl-lactose prevents alterations of the tear film induced by atropine.

As it is well known that an increase in tear osmolarity damages the eye surface, tear osmolarity was evaluated. Instillation of atropine causes a significant increase in tear osmolarity, which is prevented to a dose-dependent extent by topical ocular treatment with 2'-fucosyl-lactose (Table 2).

The results of the present study demonstrate that 2'-fucosyl lactose eyedrops are an effective treatment for dry eye syndrome. 2'-fucosyl lactose improves the integrity of the eye surface, increases the tear volume and restores the physiological tear osmolarity.

**Table 1. Schirmer's test and tear break up time (TBUT) 24 hours after first application of atropine.**

| Schirmer's test (mm) | | | | |
|---|---|---|---|---|
| No atropine | Atropine | | | |
| Control | Carrier | 0.01% 2'-fucosyl-lactose | 0.1% 2'-fucosyl-lactose | 1% 2'-fucosyl-lactose |
| 12.5±0.5 | 3.75±0.5 | 5.25±0.5* | 7.75±0.9* | 10.75±0.9* |

| TBUT (sec) | | | | |
|---|---|---|---|---|
| No atropine | Atropine | | | |
| Control | Carrier | 0.01% 2'-fucosyl-lactose | 0.1% 2'-fucosyl-lactose | 1% 2'-fucosyl-lactose |
| >60 | 4.75±0.9 | 8.75±0.9* | 18.5±1.0* | 34.5±1.3* |

| | | | | |
|---|---|---|---|---|
| *p<0.05 *vs.* carrier; n=6-8 | | | | |

**Table 2. Osmolarity of tears 24 hours after first application of atropine.**

| Tear osmolarity (mOsm/L) | | | | |
|---|---|---|---|---|
| No atropine | Atropine | | | |
| Control | Carrier | 0.01% 2'-fucosyl-lactose | 0.1% 2'-fucosyl-lactose | 1% 2'-fucosyl-lactose |
| 335±12 | 428±36 | 349±29* | 330±18* | 322±14* |

| | | | | |
|---|---|---|---|---|
| *p<0.05 *vs.* carrier; n=6-8 | | | | |

### Bibliography

Aragona, P., Giuffrida, S., Di Stefano, G., Ferreri, F., Di Benedetto, A., Bucolo, C., et al. (2002). Ocular surface changes in type 1 diabetic patients. Adv Exp Med Biol. 506, 667-72.
Chaumeil, C., Liotet, S., Kogbe, O. (1994). Treatment of severe eye dryness and problematic eye lesions with enriched bovine colostrum lactoserum. Adv. Exp. Med. Biol. 350, 595-599.
Coyne, M.J., Reinap, B., Lee, M.M., Comstock, L.E. (2005). Human symbionts use host-like pathway for surface fucosylation. Science 307, 1778-1781.
Lemp, M.A., Baudouin, C., Baum, J., Dogru, M., Foulks, G.N., Kinoshita, S. et al. (2007). Report of the International Dry Eye Workshop (DEWS). Ocul. Surf. 5, 75-92.
Liu, H., Begley, C., Chen, M., Bradley, A., Bonanno, J., McNamara, N.A. et al. (2009). A link between tear instability and hyperosmolarity in dry eye. Invest. Ophthalmol. Vis. Sci. 50, 3671-3679. doi: 10.1167/iovs.08-2689.
Musumeci, M., Simpore, J., D'Agata, A., Sotgiu, S., Musumeci, S. (2006). Oligosaccharides in colostrum of Italian and Burkinabe women. J. Pediatr. Gastroenterol. Nutr. 43, 372-378.
Newburg, D.S. (1996). Oligosaccharides and glycoconjugates in human milk: their role in host defense. J. Mammary Gland Biol. Neoplasia 1, 271-278.
Newburg, D.S., Ruiz-Palacios G.M., Altaye, M., Chaturvedi, P., Guerrero, M.L., Meinzen-Derr, J.K. et al. (2004). Innate protection conferred by fucosylated oligosaccharides of human milk against diarrhea in breastfed infants. Glycobiology 14: 253-263.
Okano, M., Satoskar, A.R., Nishizaki, K., Harn, D.A. Jr. (2001). Lacto-N-fucopentose III found on Schistosoma mansoni egg antigens functions as adjuvant for proteins by inducing Th2-type response. J. Immunol. 167, 442-450.
Sarkar, K., Sarkar, H.S., Kole, L., Das, P.K. (1996). Receptor-mediated endocytosis of fucosylated neoglycoprotein by macrophages. Mol. Cell Biochem. 156, 109-16.
Shafiee, A., Bucolo, C., Budzynski, E., Ward, K.W., López, F.J. (2011). In vivo ocular efficacy profile of mapracorat, a novel selective glucocorticoid receptor agonist, in rabbit models of ocular disease. Invest. Ophthalmol. Vis. Sci. 52, 1422-30. doi: 10.1167/iovs.10-5598.
Sotgiu, S., Arru, G., Fois, M.L., Sanna, A., Musumeci, M., Rosati, G. et al. (2006). Immunomodulation of fucosyl-lactose and lacto-N-fucopentaose on mononuclear cells from multiple sclerosis and healthy subjects. Int. J. Biomed. Sci. 2:114-120.
Smith, J.A., Albeitz, J., Begley, C., Caffery, B., Nichols, K., Schaumberg, D. et al. (2007). The epidemiology of dry eye disease: report of the Epidemiology Subcommittee of the International Dry Eye WorkShop (DEWS). Ocul. Surf. 5, 93-107.

## Claims

1. A pharmaceutical composition for topical ophthalmic administration comprising as an active ingredient 2'-fucosyl-lactose and at least one pharmaceutically acceptable carrier, adjuvant and/or diluent.

2. A composition according to claim 1, in the form of a liquid, gel, cream, lotion, liposome, suspension or eyedrop.

3. A composition according to claim 1 or 2 further comprising hyaluronic acid or salts thereof as carrier.

4. A composition according to claims 1-3 for use in the treatment of dry eye syndrome or ocular inflammation.

5. A composition for use according to claim 4 wherein the ocular inflammation derives from alterations of the tear film.

## Patentansprüche

1. Pharmazeutische Zusammensetzung für die topische Verabreichung am Auge, die 2'-Fucosyllactose als Wirkstoff und wenigstens einen pharmazeutisch annehmbaren Träger, Adjuvans und/oder Verdünnungsmittel umfasst.

2. Zusammensetzung gemäß Anspruch 1 in Form einer Flüssigkeit, eines Gels, einer Creme, Lotion, eines Liposoms, einer Suspension oder von Augentropfen.

3. Zusammensetzung gemäß Anspruch 1 oder 2, weiterhin umfassend Hyaluronsäure oder Salze davon als Träger.

4. Zusammensetzung gemäß Anspruch 1 bis 3 zur Verwendung bei der Behandlung von Keratoconjunctivitis sicca oder Augenentzündung.

5. Zusammensetzung zur Verwendung gemäß Anspruch 4, wobei die Augenentzündung durch Veränderungen des Tränenfilms verursacht ist.

## Revendications

1. Composition pharmaceutique pour une administration ophtalmique par voie locale comprenant, à titre d'ingrédient actif, du 2'-fucosyl-lactose et au moins un support, un adjuvant et/ou un diluant pharmaceutiquement acceptable.

2. Composition selon la revendication 1, sous la forme d'un liquide, d'un gel, d'une crème, d'une lotion, d'un liposome, d'une suspension ou d'une instillation oculaire.

3. Composition selon la revendication 1 ou 2, comprenant en outre de l'acide hyaluronique ou ses sels à titre de support.

4. Composition selon les revendications 1 à 3, pour son utilisation dans le traitement du syndrome de sécheresse oculaire ou d'une inflammation oculaire.

5. Composition pour son utilisation selon la revendication 4, dans laquelle l'inflammation oculaire dérive d'altérations du film lacrymal.
